# FASCICULE DE BREVET EUROPEEN

(11) **EP 1 556 466 B1**
(45) Date de publication et mention de la délivrance du brevet: **16.09.2009**
(21) Numéro de dépôt: 03780256.8
(22) Date de dépôt: 30.10.2003
(51) Int. Cl.: C11D 3/386, C11D 3/00, C11D 11/00

(54) **PROCEDE D ELIMINATION DU BIOFILM**
VERFAHRUNG ZUR ENTFERNUNG VON BIOFILM
METHOD FOR ELIMINATING BIOFILM

(30) Priorité: 31.10.2002 FR 0213963
(43) Date de publication de la demande: 27.07.2005
(73) Titulaire: Marion, Karine, 01700 Saint Maurice de Beynost (FR); Sanchez, Thierry, 01700 Saint Maurice de Beynost (FR)
(72) Inventeur: MARION, Karine, F-01700 Saint Maurice de Beynost (FR)
(74) Mandataire: Tripoz, Inès
(86) Numéro de dépôt international: PCT/FR2003/003246
(87) Numéro de publication internationale: WO 2004/041988

(56) Documents cités:
- EP-A- 0 590 746
- WO-A-01/53010
- WO-A-92/13807
- WO-A-96/40854
- FR-A- 2 102 851
- US-A- 4 609 493

## Description

La présente invention a trait au domaine de la désinfection et de la décontamination de matériel et d'appareils présentant des surfaces susceptibles de servir de support au dépôt d'un biofilm.
Ce matériel et/ou ces appareils sont par exemple ceux utilisés dans le domaine médical, comme les appareils d'analyse et tout le matériel comme les dispositifs médicaux réutilisables, comme les générateurs de dialyse ainsi que les implants (implants oculaires, valves cardiaques) et prothèses. Le matériel utilisé en dntisterie, voire les muqueuses et les dents elles-mêmes naturelles ou prothétiques sont susceptibles également d'être le siège de dépôt de biofilm. Le matériel utilisé dans l'industrie alimentaire et ou pharmaceutique peut également être cité ainsi que les centrales de climatisation et de manière plus générale tout matériel en contact avec un milieu hydrique susceptible de contenir des bactéries en suspension.
Le problème majeur actuel est l'élimination du biofilm constitué par la biomasse fixée sur les surfaces du matériel, des appareils et ou des muqueuses et qui constitue une cause d'infections persistantes et ou de contamination. En effet toute bactérie en suspension dans un milieu hydrique a la propriété d'adhérer aux supports qu'elle rencontre pour former un biofilm. Ce biofilm est un agglomérat de bactéries sur une surface entourées d'une matrice d'exopolysaccharides, sa formation est un phénomène naturel. Une fois formé le biofilm est très difficile à éliminer.
Les procédés actuels de décontamination et/ou de désinfection proposés pour lutter contre le biofilm, bien qu'ayant une certaine efficacité notamment antibactérienne n'éliminent cependant pas le biofilm du support, ce qui favorise son redéveloppement, et dans le cas particulier de l'hémodialyse, laisse en surface des supports des pyrogènes.
Pour certains appareils ou matériels le biofilm est en outre renforcé par des dépôts de tartre constitués de carbonate de calcium et ou de magnésium.
L'utilisation actuelle de solutions décalcifiantes en renforcement des solutions purement désinfectantes pour le traitement de certain matériel ne permet cependant pas l'élimination totale de ce biofilm sauf à utiliser des produits comme l'eau de Javel qui si elles sont efficaces sur le biofilm sont souvent destructrices pour le matériel et les appareils médicaux. Ces solutions sont en outre inutilisables sur des surfaces en contact avec das tissus et ou directement sur les muqueuses.
On connait de Jacquelin L.F.. Pathologie Biologie, mai 1994, p. 425 l'utilisation sequentielle d'enzymes et de désinfectant phénolique pour la destruction des biofilms. On connaît de Johansen C., Applied and Environmental Microbiology septembre 1997, p. 3724, l'utilisation de combinaisons enzymatiques comme les glucose oxydases et la lactoperoxydase. On connaît de WO01/53010 un procédé enzymatique d'élimination des biofilms comprenant l'utilisation d'une enzyme appartenant au groupe des carbohydrases, et des protéases et leur et également, en combinaison ou de façon indépendant d'argents appartenant au groupe des biocides, chélatans, et autres nettoyants. WO 92/13807 décrit également des compositions et procédés pour éliminer ou prévenir la formation de biofilm dans un système d'eau industrielle par mise en contact d'une protéase, d'une amylase et d'un tensio-actif, cette mise en contact étant simultanée ou séquentielle.

On connaît également de EP1186574, un procédé d'élimination des biofilms des surfaces en contact avec de l'eau caractérisé en ce qu'il comporte un nettoyage avec un principe actif enzymatique et un nettoyage avec un produit désinfectant destiné à tuer les bactéries libérées par l'action du mélange enzymatique, mais les résultats obtenus ne sont pas satisfaisants et aucun procédé, ni produit ne permet actuellement d'éliminer ces biofilms.

La présente invention permet de résoudre le problème par la mise en oeuvre d'un procédé et par une sélection de produits permettant d'obtenir une efficacité d'élimination jamais obtenue jusqu'à présent.

la présente invention concerne un procédé d'élimination biofilm caractérisé en ce qu'il comprend au moins les étapes suivantes effectuées simultanément ou consécutivement
a) on dispose d'une solution comprenant un mélange enzymatique contenant au moins une enzyme choisie dans le groupe des protéases, au moins une enzyme choisie: dans le groupe des estérases et une amylase.
b) on dispose d'une solution comprenant un détergent dont le pH est alcalin,
c) on applique par lavage ou par circulation lesdites solutions sur la surface à traiter.

Dans une variante, le procédé selon l'invention comprend en outre les étapes suivantes effectuées simultanément ou consécutivement :
d) on dispose d'une solution comprenant un acide susceptible de dissoudre des dépôts de sels minéraux,
e) on applique par lavage ou par circulation ladite solution sur la surface à traiter.

L'invention concerne également :
- le procédé selon l'invention, caractérisé en ce que en ce que l'enzyme choisie dans le groupe des protéases est choisie dans le groupe constitué par les exopeptidases ou les endopeptidases comme la trypsine ;
- le procédé selon l'invention, caractérisé en ce que l'enzyme choisie dans le groupe des estérases est une carboxylester-hydrolase comme la lipase, une phospholipase et/ou une phosphonodiestérase comme la ribonucléase ;
- le procédé selon l'invention, caractérisé en ce que en ce que le mélange enzymatique comprend en outre une enzyme choisie dans le groupe constitué par les osidases ou carbohydrases comme la glycosidase et la galactosidase ;
- le procédé selon l'invention, caractérisé en ce que le mélange enzymatique est la pancréatine ;
- le procédé selon l'invention, caractérisé en ce que le détergent est une solution alcaline contenant des agents tensioactifs ;
- le procédé selon l'invention, caractérisé en ce que le détergent est une solution alcaline contenant des agents tensioactifs et un ammonium quaternaire ;
- le procédé selon l'invention, caractérisé en ce que le détergent contient en outre un désinfectant comme une solution d'hypochlorite de sodium, ou d'hypochlorite de potassium.

Lorsque dans une variante le procédé comprend une étape de lavage par une solution acide pour éliminer les dépôts de sels minéraux, l'acide est choisi dans le groupe constitué par l'acide citrique, l'acide peracétique, l'acide glycolique et l'acide hydroxyacétique.

L'invention concerne également un kit destiné à l'élimination du biofilm caractérisé en ce qu'il comprend au moins une solution d'un mélange enzymatique contenant au moins une enzyme choisie dans le groupe des protéases, au moins une enzyme choisie dans le groupe des estérases et une amylase, et au moins une solution d'un détergent dont le pH est alcalin.

L'invention concerne également :
- un kit selon l'invention, caractérisé en ce que l'enzyme choisie dans le groupe des protéases est choisie dans le groupe constitué par les exopeptidases ou les endopeptidases comme la trypsine ;
- un kit selon l'invention, caractérisé en ce que l'enzyme choisie dans le groupe des estérases est une carboxylester-hydrolase comme la lipase, une phospholipase et/ou une phosphonodiestérase comme la ribonucléase ;
- un kit selon l'invention, caractérisé en ce que le mélange enzymatique comprend en outre une enzyme choisie dans le groupe constitué par les osidases ou carbohydrases comme la glycosidase et la galactosidase ;
- un kit selon l'invention, caractérisé en ce que le mélange enzymatique est la pancréatine ;
- un kit selon l'invention, caractérisé en ce le détergent est une solution alcaline contenant des agents tensioactifs ;
- un kit selon l'invention caractérisé en ce que le détergent est une solution alcaline contenant des agents tensioactifs et un ammonium quaternaire ;
- un kit selon l'invention, caractérisé en ce qu'il comporte en outre une solution d'un désinfectant comme une solution d'hypochlorite de sodium, ou d'hypochlorite de potassium ;
- un kit selon l'invention, caractérisé en ce qu'il comporte en outre une solution d'un acide susceptible de dissoudre des dépôts de sels minéraux comme le carbonate de calcium ;
- un kit selon l'invention, caractérisé en ce que dans la solution acide pour éliminer les dépôts de sels minéraux, l'acide est choisi dans le groupe constitué par l'acide citrique, l'acide peracétique, l'acide glycolique et l'acide hydroxyacétique.

Le mélange enzymatique est de préférence utilisé à une concentration comrise entre 0,1 et 10 % en poids/volume ; le détergent est de préférence utilisé à une concentration comprise entre 0,1 et 30 % en volume/volume, et lorsque celui-ci comprend en outre un désinfectant, le désinfectant est additionné de préférence à une concentration de 0.01 à 2%.

La durée d'application selon le procédé de l'invention de la solution comprenant le mélange enzymatique est comprise entre 5 mn et une heure, en fonction du type de biofilms, de son ancienneté et du matériau : cette application est de préférence effectuée à une température comprise entre la température ambiante et 40 °C, et de préférence à 37 °C.
La solution comprenant un détergent dont le pH est alcalin est appliquée pendant une durée qui peut varier de 5 mn à 24 heures, à une température comprise entre la température ambiante et 90 °C, en fonction du type de biofilm et du matériel traité.

L'invention concerne également une composition destinée. à l'élimination du biofilm caractérisée en ce qu'elle comprend un mélange enzymatique contenant au moins une enzyme choisie dans le groupe des protéases, au moins une enzyme choisie dans le groupe des estérases et une amylase, et un détergent dont le pH est alcalin.
Plus particulièrement la composition selon l'invention est caractérisée en ce que le mélange enzymatique est la pancréatine.

Dans une variante de l'invention la solution comprenant le mélange enzymatique et ta solution comprenant le détergent forme une solution unique ; l'invention concerne donc également une composition destinée à l'élimination du biofilm caractérisée en ce qu'elle comprend un mélange enzymatique contenant au moins une enzyme choisie dans le groupe des protéases, au moins une enzyme choisie dans le groupe des estérases et une amylase, et un détergent dont le pH est alcalin.

Dans un mode de réalisation particulier le procédé selon l'invention est caractérisé en ce que le détergent est une solution neutre ou une solution acide contenant des agents tensioactifs : dans cette variante lorsque le détergent est une solution acide il permet sans étape supplémentaire de dissoudre les dépôts de sels minéraux, lorsque le phénomène d'entartrage est très important.

On entend par « biofilm », un ensemble de microorganismes développés sur un support, notamment bactéries, virus, parasites et champignons, Ce biofilm se développe et les microorganismes sécrètent une gangue d'exopolymères contenant entre autre des exopolysaccharides qui va former un film biologique appelé « slime » ou « glycocalix » et qui se présente sous forme d'un dépôt gélatineux à la surface des parois.
On entend par « pancréatine », un extrait de pancréas, contenant l'ensemble des enzymes digestives de cette glande, notamment des enzymes protéolytiques ou protéases et des hydrolases notamment les estérases comme la lipase, de l'amylase, de la ribonucléase et de la trypsine, on se référera à la définition de la Pharmacopée Européenne.
On entend par « détergent » tout produit dont la composition a été spécialement étudiée pour concourir au développement des phénomènes de détergence et qui comprend comme composants essentiels les agents de surface qui sont des tensio-actifs et éventuellement des composants complémentaires (adjuvants, renforçateurs, charges, additifs divers). Les tensio-actifs sont des composés chimiques qui introduits dans un liquide, en abaissent la tension superficielle, ce qui a pour effet d'en augmenter les propriétés mouillantes.
On entend par « pH alcalis » une solution aqueuse présentant un pH supérieur à 7, et de préférence dans la présente invention un pH supérieur ou égal à 9.
On entend par « élimination du biofilm », le décrochage du biofilm de son support.

L'efficacité du procédé selon l'invention a été testée selon le plan d'expérience décrit ci-après.

Le procédé a été testé et mis en oeuvre expérimentalement sur 5 types de biofilms ; les biofilms 1, 2, 3 et 5 ont été obtenus grâce à un modère in vitro mimant le générateur d'hémodialyse.
Biofilm1 : enrichi en nutriments de croissance accélérée (3 jours) d'épaisseur moyenne (environ 10⁵ UFC/cm²), très riche en « slime »
Biofilm 2 : non enrichi en nutriments, s'étant développé en 1 mois. équivalent à ceux rencontrés réellement dans les générateurs de dialyse (environ 10³ UFC/cm²), très riche en cristaux de tartre
Biofilm 3 : enrichi en nutriments de croissance accélérée (5 jours) épais (environ 10⁹ UFC/cm²), très riche en « slime »
Biofilm 4 : échantillon de tubulure véhiculant de l'eau pour hémodialyse, prélevé dans un centre, recouvert d'un biofilm d'environ 10³ UFC/cm² mais s'étant développé en plus d'un an.
Biofilm 5 : enrichi en nutriments de croissance accélérée, s'étant développé dans un modèle « préventif » en 3 semaines.

### Réalisation du modèle in vitro

Un corps de réacteur de 250 ml a été rempli par un dialysat non stérile, préparé par dilution de solutions concentrées pour hémodialyse stériles apyrogènes (Clearflex®, Bieffe Medital), avec de l'eau osmosée non stérile contenant *Pseudomonas putida, Pseudomonas fluorescens, Flavimonas orizibitans,* produite en continu au laboratoire.
Le milieu contaminant circulait en circuit fermé dans une boucle de tubulure en silicone de 1,5 mètres de long et de 5 mm de diamètre interne à un débit de 500 ml/min grâce à une pompe péristaltique. L'ensemble des tubulures et le corps de réacteur ont été préalablement stérilisés à l'autoclave à 121°C pendant 30 minutes. Ainsi, le dialysat contaminé naturellement par les bactéries de l'eau était la seule source de micro-organismes.
L'ensemble du système a été maintenu à une température de 37°C grâce à une plaque chauffante sur laquelle était posé le corps de réacteur.
Dans le cas des biofilms 1, 3 et 5 la croissance bactérienne et par conséquent le développement du biofilm ont été accélérés par ajout de bouillon de culture LB dilué au 1/50 ^{ème}, soit une solution de bouillon LB diluée au 1/5^{ème} apportée à un débit égal au 1/10^{ème} de celui du dialysat.Les débits d'apport du dialysat et du bouillon de culture, régulés par une pompe péristaltique, étaient respectivement de 5 et 0,5 ml/minute.
Dans le cas du biofilm 5, le modèle a été modifié de la façon suivante :
Des segments de tubulures en silicone connectés entre-eux par des raccords en polypropylène, ont été parcourus pendant 4 heures par un dialysat non stérile enrichi en milieu de culture. Toutes les 4 heures, les tubulures ont été déconnectées et intégrées à des systèmes de désinfection (voir ci -après) Après traitement, les tubulures ont été reconnectées et le milieu contaminant a été remis à circuler pendant 4 heures. En parallèle, des tubulures témoins ont été réparties dans le circuit et sans jamais subir de désinfection. Chaque jour, 2 séances d'hémodialyse entrecoupées chacune d'une séance de désinfection ont ainsi pu être effectuées. La nuit et les weekend, le système était arrêté après la dernière désinfection et les tubulures étaient maintenues vides à température ambiante. Le système a fonctionné jusqu'au développement d'un biofilm mature sur les tubulures témoins.

### Produits et combinaisons testés

17 produits ont été testés appartenant à 6 familles différentes. La liste de ces produits est donnée dans le tableau I. Ces produits ont été évalués seuls ou en combinaisons. Ainsi, un screening complet de 60 combinaisons a été réalisé sur le biofilm 1 ; 9 - combinaisons ont ensuite été évaluées sur le biofilm 2 ; enfin, la meilleure combinaison sélectionnée a été testée sur les biofilms 3, 4 et 5.

**Tableau I : Liste des produits testés**

| **Famille** | **Produit** | **Fournisseur** |
|---|---|---|
| Surfactants /détergents | Sodium dodecyl sulfate | Sigma |
| | Triton | Sigma |
| | RBS | Chemical Products |
| | Tween | Sigma |
| Enzymes | Trypsine | Sigma |
| | Pancréatine | Sigma |
| | Protéase fongique | Sigma |
| | Thermolysine | Sigma |
| Acides | Acide perchlorique | Merck |
| | Acide citrique | Merck |
| | Acide trichloracétique | Merck |
| Produits de dissociation cellulaire | Versène | Sigma |
| | Cell dissociation | Sigma |
| Alcalins | NaOH | Prolabo |
| | KOH | Prolabo |
| Divers | Eau de Javel | - |
| | Tampon pH10 (Bicarbonate) | Préparé au labo |

### Echantillonnage

Les tubulures recouvertes des biofilms 1, 2, et 3 ont été découpées en segments de 5 cm de long. Pour le screening sur les biofilms 1 et 2, chaque segment a été sélectionné par tirage au sort pour subir l'un des différents traitements à étudier. Des échantillons contrôle prélevés au hasard sur la boucle en silicone ont été conservés non traités

### Traitement des échantillons

Les segments de tubulure à traiter ont été fixés sur la tubulure descendante d'un montage constitué de 2 tubulures, l'une ascendante, l'autre descendante, d'une pompe péristaltique et d'un bain marie (pour les traitements effectués à une température supérieure à 20°C). Le produit à tester en mode « recirculation »a été mis en solution dans une fiole de 100 ml et a été entraînée par la pompe péristaltique à un débit de 500 ml/min en circuit fermé à travers les tubulures pendant une durée respectant les temps de contact décrits dans le tableau II. Le produit à tester en mode « statique » a été mis en solution dans une fiole de 100 ml et a été entraînée par la pompe péristaltique jusqu'à remplissage des tubulures ; puis, la pompe a été arrêtée et le produit maintenu en stase pendant le temps de contact souhaité. Après chaque traitement par un produit donné, les échantillons de tubulures ont été rincés 5 minutes par de l'eau osmosée.

### Méthodes d'étude de l'efficacité des traitements

Trois paramètres fondamentaux ont été retenus pour l'évaluation de l'efficacité des traitements
la réduction de la surface couverte
la réduction du nombre de bactéries cultivables
la réduction du taux d'endotoxines

Les screening sur les biofilms 1 et 2 n'ont tenu compte que du premier paramètre. La meilleure combinaison retenue a ensuite été évaluée de manière approfondie pour son efficacité sur la mortalité bactérienne et l'élimination des endotoxines.

### Méthode d'évaluation quantitative de la surface couverte :

Les biofilms témoins et traités ont été colorés:
- soit par une solution de cristal violet à 0.25%
- soit par une solution de fluorochromes Baclight® (Syto 9 et iodure de propidium). Les bactéries viables apparaissent vertes ; les mortes apparaissent jaunes ou rouges.

Les échantillons de tubulure en silicone recouverte de biofilm coloré ont été attachés sur des lames de verre et observés au microscope optique, relié lui-même à une caméra et à un logiciel d'analyse d'images « Scion Images ». Ainsi. plusieurs photographies d'un même échantillon (6 à 10 ) ont été prises, la surface colorée a été évaluée quantitativement par le logiciel d'analyse d'images, et une valeur moyenne de surface couverte par échantillon a été calculée. Cette valeur moyenne a été comparée à la surface couverte des échantillons témoins non traités : un pourcentage de réduction de la surface couverte a alors été calculé.
D'autre part, pour une observation plus précise, les échantillons traités par la combinaison la plus efficace ont été observés au microsocpe confocal laser.

### Méthode de quantification des bactéries cultivables

Le biofilm recouvrant les échantillons de tubulure a été détaché du support par un grattoir mécanique assurant un décrochage complet, homogène et reproductible. Ce grattoir était constitué d'un tournevis électrique à l'extrémité duquel était fixée une spatule en acier inoxydable stérilisable à la flamme. La rotation de la spatule dans la lumière de la tubulure a entraîné la biomasse au fond d'un tube stérile. L'entraînement a été facilité par un filet d'eau stérile. Les éventuels agrégats bactériens ont ensuite été dissociés au travers de l'aiguille d'une seringue. Le nombre de bactéries cultivables a été déterminé par dénombrement des UFC après étalement de la suspension bactérienne résultante sur gélose R₂A incubée à température ambiante pendant 7 jours.

### Méthode de dosage des endotoxines

Les endotoxines bactériennes ont été quantifiées dans la suspension bactérienne résultant du décrochage (voir ci-dessus) par les test standardisé de référence : le test LAL chromogénique cinétique (Charles River Endosafe).

### Résultats de l'étude sur les biofilms 1 et 2

Les résultats des screening sur les biofilms 1 et 2 sont présentés dans les tableaux II et III

**Tableau II : Screening sur le Biofilm 1**

| **Trait^{t} n°** | **produit** | **conc** | **°C** | **temps** | **mode** | **résultat** |
|---|---|---|---|---|---|---|
| 1 | SDS | 5% | amb | 40 min | stat | ** |
| 2 | triton | 5% | amb | 40 min | stat | * |
| 3 | RBS | 5% | amb | 40 min | stat | ** |
| 4 | tween | 5% | amb | 40 min | stat | . |
| 5 | versene | pure | amb | 40 min | stat | . |
| 6 | trypsin | EDTA IX | amb | 40 min | stat | . |
| 7 | trypsin | 0,25% | 37 | 40 min | stat | ** |
| 8 | SDS | 5% | amb | 40 min | recirc | */. |
| 9 | tween | 5% | amb | 40 min | recirc | . |
| 10 | RBS | 5% | amb | 40 min | recirc | *** |
| 11 | perchlo ac | 0,05% | amb | 40 min | stat | . |
| 12 | NaOH | 0.01 N | amb | 40 min | stat | * |
| 13 | KOH | 0.02N | amb | 40 min | stat | ** |
| 14 | TCA | 0.25% | amb | 40 min | stat | . |
| 15 | KOH | 0.02N | amb | 40 min | recirc | ** |
| 16 | triton | 5% | amb | 40 min | recirc | . |
| 17 | RBS | 5% | amb b | 1h | recirc | *** |
| 18 | RBS | 5% | amb | 24h | recirc | **** |
| 19 | RBS | 2% | amb | 1 h | recirc | *** |
| 20 | RBS | 2% | amb | 24h | recirc | **** |
| 21 | KOH | 0.02N | amb | 1 h | recirc | *** |
| 22 | KOH | 0.02N | amb | 24h | recirc | **** |
| 23 | pancreatin | 1 % | 37 | 1 h | recirc | **** |
| 24 | pancreatin | 1 % | 37 | 1h | stat | ** |
| 25 | pancreatin | 0,10% | 37 | 2h | recirc | . |
| 26 | citric ac | 3% | amb | 40 min | recirc | . |
| 27 | KOH | 0.002N | amb | 40 min | recirc | ** |
| 28 | Cell dissoc | pure | 37 | 40 min | stat | ** |
| 29 | trypsin | 0,25% | 37 | 40 min | recirc | . |
| 30 | protease | 0,25% | 37 | 40 min | recirc | ** |
| 31 | RBS | 2% | amb | 40 min | recirc | **** |
| 32 | RBS +Cl | 2%+0.2% | amb | 40 min | recirc | **** |
| 33 | thermolys | 2mg/50ml | 37 | 40 min | recirc | ** |
| 34 | pancreatin | 0,50% | 37 | 40 min | recirc | *** |
| 35 | KOH | 0.001 N | amb | 40 min | recirc | ** |
| 36 | RBS | 2%ph7 | amb | 40 min | recirc | *** |
| 37 | trypsin | 1 % | 37 | 40 min | recirc | * |
| 38 | protease | 1 % | 37 | 40 min | recirc | ** |
| 39 | RBS | 1% ph10 | amb | 40 min | recirc | **** |
| 40 | RBS | 0.5%ph10 | amb | 40 min | recirc | ***(*) |
| 41 | RBS | 1% ph10 | amb | 5 min | recirc | *** |
| 42 | RBS | 0.1%ph10 | amb | 40 min | recirc | **(*) |
| 43 | RBS + pancreatin | 1% ph10 1 % | 40 | 5 min | recirc | * |
| 44 | RBS + pancreatin | 1% ph10 0,50% | 40 | 5 min | recirc | ** |
| 45 | RBS + pancreatin | 1% ph10 0,50% | 40 | 40 | recirc | *** |
| 46 | buffer ph10 | pure | 37 | 40 | recirc | * |
| 47 | pancreatin | 0.5 ph10 | 37 | 40 | recirc | ** |
| 48 | pancreatin et RBS | 0.5%ph7 1% ph10 | 37 amb | 5 min 5 min | recirc recirc | ***** |
| 49 | pancreatin | 0.25%ph7 | 37 | 5min | recirc | *** |
| 50 | pancreatin + thermolys | 0.25%ph7 2mg/50ml | 37 | 5min | recirc | ** |
| 51 | pancreatin + thermolys + protease | 0.25%ph7 2mg/50ml 0,25% | 37 | 5min | recirc | ** |
| 52 | pancreatin + thermolys + protease + trypsin | 0.25%ph7 2mg/50ml 0,25% 0,25% | 37 | 5min | recirc | ** |
| 53 | pancreatin et RBS | 0,25 1% | 37 amb | 5 min 5 min | recirc | ***** |
| 54 | pancreatin et RBS | 0,25% 0,50% | 37 amb | 5 min 5 min | recirc | ***** |
| 55 | pancreatin et RBS | 0,25% 0,50% | 37 amb | 5 min 30 min | recirc | ***** |
| 56 | pancreatin et RBS | 0,10% 0,50% | 37 amb | 5 min 5 min | recirc | ***** |
| 57 | pancreatin et RBS | 0,10% 0,10% | 37 amb | 5 min 5 min | recirc | ***** |
| 58 | citric ac et RBS | 3% 1% | 37 amb | 5 min 30 min | recirc | ***** |
| 59 | citric ac et pancreatin et RBS | 3% 0,50% 1 % | 37 37 amb | 5 min 5 min 30 min | recirc | ***** |
| 60 | pancreatin et RBS | 0,50% 1 % | 37 amb | 5 min 30 min | recirc | ***** |

| Légende | **Elimination** | **% réduction surface couverte** |
|---|---|---|
| . | Pas 'élimination | 0 |
| * | Mauvaise élimination | 0-25% |
| ** | Elimination modérée | 25-50% |
| *** | Bonne élimination | 50-75% |
| **** | Excellente élimination | 75-99% |
| ***** | Elimination complète | 100% |

| | | |
|---|---|---|
| + = mélange et = application séquentielle | | |

**Tableau III : Screening sur le biofilm 2**

| **Trait^{t} n°** | **produit** | **conc** | **°C** | **temps** | **mode** | **résultat** |
|---|---|---|---|---|---|---|
| 1 | Pancreatin et RBS | 0.5% ph7 1% ph10 | 37 amb | 5 min 5 min | recirc recirc | *** |
| 2 | citric ac et RBS | 3% 1% | 37 amb | 5 min 30 min | recirc | ***** |
| 3 | pancreatin et citric ac et RBS | 0,50% 3% 1% | 37 37 amb | 5 min 5 min 30 min | recirc | ***** |
| 4 | pancreatin et RBS | 0,50% 1% | 37 amb | 5 min 30 min | recirc | *** |
| 5 | citric ac et pancreatin et RBS | 3% 0,50% 1 % | 37 37 amb | 5 min 5 min 30 min | recirc | ***** |
| 6 | pancreatin et RBS | 0,50% 1 % | 37 amb | 5 min 30 min | recirc | *** |
| 7 | citric ac | 3% | 37 | 5 min | recirc | **** |
| 8 | citric ac et RBS | 3% 0,50% | 37 amb | 5 min 30 min | recirc | ***** |
| 9 | citric ac et RBS | 3% 0,10% | 37 amb | 5 min 30 min | recirc | **** |

La combinaison retenue suite à ces screening a été celle permettant la meilleure élimination des 2 biofilms, à savoir, la « combinaison K » suivante :

*Produit A* = Pancreatin^{®}, réactif de laboratoire commercialisé par Sigma. Extrait du pancréas de porc, il s'agit d'un mélange enzymatique contenant entre autre lipase, protéase, amylase, trypsine, ribonuclease (voir Pharmacopée Européenne).

*Produit B* = acide citrique
Dans le cas particulier de la désinfection des générateurs de dialyse, ce produit agit comme décalcifiant et élimine les cristaux de tartre qui emprisonnent les bactéries et favorisent l'adhésion du biofilm au support.

*Produit C* = RBS^{®} , solution détergente alcaline moussante, commercialisée par la société Chemical Products, à propriétés bactéricides, virucides et fongicides, contenant des agents tensio-actifs et un ammonium quaternaire (agent désinfectant).

### Données qualitatives et quantitatives

Des photographies des biofilms 1 et 2 avant et après action de la combinaison K ont permis de quantifier visuellement l'action de la combinaison.
Le tableau IV donne les valeurs des paramètres mesurés avant et après action de la combinaison K.

**Tableaux IV :** Données quantitatives de l'évaluation de l'efficacité de la combinaison K sur les biofilms 1 et 2

**Tableau IV a) Biofilm 1**

| **Paramètre** | **Avant traitement** | **Après traitement** | **% réduction** |
|---|---|---|---|
| Surface couverte (Inches ²) | 20 | <0,001 | >99,99 |
| Bactéries cultivables (UFC/cm²) | 10⁵ | <1 | >99,999 |
| Endotoxines(EU/cm²) | 10039 | <0,005 | >99,99 |

**Tableau IV b) Biofilm 2**

| **Paramètre** | **Avant traitement** | **Après traitement** | **% réduction** |
|---|---|---|---|
| Surface couverte (Inches²) | 13,4 | <0,001 | >99,99 |
| Bactéries cultivables (UFC/cm²) | 3.10³ | <1 | >99,999 |
| Endotoxines(EU/cm²) | 40 | <0,005 | >99,99 |

### Détermination de la MIC du RBS

Le pouvoir antibactérien de cette combinaison étant porté par le RBS, sa concentration minimale inhibitrice a été déterminée *sur les germes constituant les biofilms étudiés.*
Un mélange de dialysat frais contaminé (préparé avec de l'eau osmosée non stérile contenant les germes décrits précédemment) et de bouillon LB dans les proportions 50/50 v/v a été réalisé. Des solutions de RBS aux concentrations de 100 %, 50 %, 10 %, 5 %, 1 %, 0,5 % et 0,1 % ont été faites par dilutions en cascades, puis 300 µl de chacune de ces solutions ont été ajoutés à 3 ml du mélange contaminé. Après 12 heures d'incubation à température ambiante, les UFC ont été dénombrées sur gélose R₂A pour chacune des concentrations de RBS testées.

La MIC est définie comme étant la plus faible concentration qui conduit à l'inhibition de la croissance des germes. Les résultats sont donnés dans le tableau V.

**Tableau V : Détermination de la MIC du RBS**

| | | | | | | | | | | |
|---|---|---|---|---|---|---|---|---|---|---|
| Conc (%) | 0 | 0,01 | 0,05 | 0,5 | 1 | 2 | 3 | 4 | 5 | 10 |
| UFC/ml | 210⁸ | 4,8 10⁷ | 2,1 10⁷ | 9 10⁶ | 6,1 10⁶ | 1,4 10⁵ | 1200 | 200 | 0 | 0 |

Par sécurité, on choisit d'utiliser la solution de RBS diluée de manière à obtenir 1,5 MIC.

### Protocole initial retenu

Pancréatine 0,5 %, pH 7,3 : Préparation pour 100 ml : 500 mg de poudre de Pancréatine + 1 g de tampon PBS (phosphate buffer saline) (Sigma) pulvérisé, dilués dans 100 ml d'eau pour hémodialyse (EHD).
Passage de la solution en circuit fermé dans les tubulures à un débit de 500 ml/min pendant 5 minutes à 37°C.
Rinçage 5 minutes par de l'EHD à 500ml/min en circuit ouvert.

Acide citrique 3%, pH 2,2 : Préparation pour 100 ml : 3 g d'acide citrique pulvérisé (Merck) dans 100 ml d'EHD.

Passage de la solution en circuit fermé dans les tubulures à un débit de 500 mi/min pendant 5 minutes à 20°C
Rinçage 5 minutes par de l'EHD à 500ml/min en circuit ouvert.

RBS^{®} 7% pH 10: préparation pour 100 ml: 7 ml de solution concentrée + 566 mg de carbonate de sodium pulvérisés + 388 mg de bicarbonate de sodium pulvérisé , dilués dans QSP 100 ml d'EHD.
Passage de la solution en circuit fermé dans les tubulures à un débit de 500 ml/min pendant 30 minutes à 20°C .
Rinçage 5 minutes par de l'EHD à 500ml/min en circuit ouvert.

### Résultats de l'étude sur les biofilms 3 et 4

La combinaison K sous sa forme initiale décrite ci-dessus a laissé quelques cellules adhérentes sur les biofilms 3 et 4, particulièrement épais ou anciens. Pour l'élimination de tels biofilms, une « formule enrichie »de la combinaison K a été développée.

### Formule « enrichie » :

Pancréatine 1 %, pH 7;3 : Préparation pour 100 ml : 1 g de poudre de Pancréatine + 1 g de tampon PBS (phosphate buffer saline) (Sigma) pulvérisé, dilués dans 100 ml d'eau pour hémodialyse (EHD).
Passage de la solution en circuit fermé dans les tubulures à un débit de 500 ml/min pendant 5 minutes à 37°C.
Rinçage 5 minutes par de l'EHD à 500ml/min en circuit ouvert.

Acide citrique 5%, pH 2,2 : Préparation pour 100 ml : 5 g d'acide citrique pulvérisé (Merck) dans 100 ml d'EHD.
Passage de la solution en circuit fermé dans les tubulures à un débit de 500 ml/min pendant 30 minutes à 20°C
Rinçage 5 minutes par de l'EHD à 500ml/min en circuit ouvert.

RBS^{®} 15% pH 10: préparation pour 100 ml : 15 ml de solution concentrée + 566 mg de carbonate de sodium pulvérisés + 388 mg de bicarbonate de sodium pulvérisé + 6 ml d'eau de Javel concentrée à 5,2 % (concentration finale en hypochlorite de sodium de 0,3 %), dilués dans QSP 100 ml d'EHD
Passage de la solution en circuit fermé dans les tubulures à un débit de 500 ml/min pendant 1 nuit à 20°C
Rinçage 5 minutes par de l'EHD à 500ml/min en circuit ouvert.

### Données qualitatives et quantitatives

Des photographies du biofilm 4 avant et après action de la combinaison K enrichie ont permis de quantifier visuellement l'efficacité du procédé selon l'invention..
Le tableau VI donne les valeurs des paramètres mesurés avant et après action de la combinaison K enrichie sur le biofilm 4

**Tableaux VI : Données quantitatives de l'évaluation de l'efficacité de la combinaison K enrichie sur le biofilm 4**

| **Paramètre** | **Avant traitement** | **Après traitement** | **% réduction** |
|---|---|---|---|
| Surface couverte (Inches²) | 25 | <0,001 | >99,99 |
| Bactéries cultivables (UFC/cm²) | 1600 | <1 | >99,999 |
| Endotoxines(EU/cm²) | 115 | <0,005 | >99,999 |

### Résultats de l'étude sur le biofilm 5

Un biofilm très épais (plus de 3.10⁹ UFC/cm²) très riche en slime, très riche en endotoxines bactériennes et couvrant totalement la surface de l'échantillon (30 Inches²) s'est développé à la surface des échantillons contrôles non traités, alors que seulement quelques cellules adhérentes mortes se sont déposées en surface des échantillons traités toutes les 4 heures par le combinaison K non enrichie (formule initiale).
Les données quantitatives sont présentées dans le tableau VII.

**Tableau VII : Efficacité de la combinaison K sur le biofilm 5**

| **Paramètre** | **Sans traitement** | **Avec traitement** | **% inhibition** |
|---|---|---|---|
| Surface couverte (Inches ²) | 30 | 1,3 | 96 |
| Bactéries cultivables (UFC/cm²) | 3,9 10⁹ | <1 | >99,999 |
| Endotoxines(EU/cm²) | 65282 | 0,4 | >99,999 |

Des photographies du biofilm contrôle et des échantillons traités ont permis de vérifier l'efficacité du procédé selon l'invention.

Le procédé, le kit et la composition selon l'invention peuvent être utilisés dans les circuits des appareils d'hémodialyse, dans la lutte contre la légionellose par exemple dans les circuits d'eau chaude et les systèmes de climatisation et les tours de refroidissement, dans l'industrie agroalimentaire, dans les salles à atmosphère contrôlée ou dans les salles à atmosphères confinées, pour le nettoyage du matériel en dentisterie pour les dispositifs médicaux réutilisables et non autoclavables.

Dans les appareils de circulation de fluides, le procédé selon l'invention sera mis en oeuvre, par introduction de la ou des solutions simultanément ou séquentiellement dans les circuits dans lesquels le biofilm doit être éliminé, mise en circulation pendant une période suffisante pour permettre l'élimination du biofilm, puis purge et rinçage si nécessaire.
Pour le traitement de surfaces, plans de travail, prothèses, le procédé selon l'invention sera mis en oeuvre par application ou par trempage de la ou des solutions selon l'invention de façon séquentielle ou simultanée puis rinçage si nécessaire.

## Revendications

1. Procédé d'élimination du biofilm **caractérisé en ce qu'**il comprend au moins les étapes suivantes effectuées simultanément ou consécutivement :
a) on dispose d'une solution comprenant un mélange enzymatique contenant au moins une enzyme choisie dans le groupe des protéases, au moins une enzyme choisie dans le groupe des estérases et une amylase,
b) on dispose d'une solution comprenant un détergent dont le pH est alcalin,
c) on applique par lavage ou par circulation lesdites solutions sur la surface à traiter.

2. Procédé selon la revendication 1, **caractérisé en ce qu'**il comprend en outre les étapes suivantes effectuées simultanément ou consécutivement :
d) on dispose d'une solution comprenant un acide susceptible de dissoudre des dépôts de sels minéraux,
e) on applique par lavage ou par circulation ladite solution sur la surface à traiter.

3. Procédé selon l'une quelconque des revendications précédentes, **caractérisé en ce que** l'enzyme choisie dans le groupe des protéases est choisie dans le groupe constitué par les exopeptidases ou les endopeptidases comme la trypsine ;

4. Procédé selon l'une quelconque des revendications précédentes, **caractérisé en ce que** l'enzyme choisie dans le groupe des estérases est une carboxylester-hydrolase comme la lipase, une phospholipase et/ou une phosphonodiestérase comme la ribonucléase

5. Procédé selon l'une quelconque des revendications précédentes, **caractérisé en ce que** le mélange enzymatique comprend en outre une enzyme choisie dans le groupe constitué par les osidases ou carbohydrases comme la glycosidase et la galactosidase ;

6. Procédé selon l'une quelconque des revendications précédentes, **caractérisé en ce que** le mélange enzymatique est la ancréatine.

7. Procédé selon l'une quelconque des revendications précédentes, **caractérisé en ce que** le détergent est une solution alcaline contenant des agents tensioactifs .

8. Procédé selon l'une quelconque des revendications précédentes, **caractérisé en ce que** le détergent est une solution alcaline contenant des agents tensioactifs et un ammonium quaternaire ;

9. Procédé selon l'une quelconque des revendications précédentes, **caractérisé en ce que** la solution détergente contient en outre un désinfectant comme une solution d'hypochlorite de sodium, ou d'hypochlorite de potassium.

10. Procédé selon la revendication 2 **caractérisé en ce que** dans l'acide pour éliminer les dépôts de sels minéraux, l'acide est choisi dans le groupe constitué par l'acide citrique, l'acide peracétique, l'acide glycolique et l'acide hydroxyacétique.

11. Kit destiné à l'élimination du biofilm **caractérisé en ce qu'**il comprend au moins une solution d'un mélange enzymatique contenant au moins une enzyme choisie dans le groupe des protéases, au moins une enzyme choisie dans le groupe des estérases et une amylose, et **en ce qu'**il comprend au moins une solution d'un détergent dont le pH est alcalin.

12. Kit selon la revendication 11, **caractérisé en ce que** l'enzyme choisie dans le groupe des protéases est choisie dans le groupe constitué par les exopeptidases ou les endopeptidases comme la trypsine.

13. Kit selon l'une quelconque des revendications 11 ou 12 **caractérisé en ce que** l'enzyme choisie dans le groupe des estérases est une carboxylester-hydrolase comme la lipase, une phospholipase et/ou une phosphonodiestérase comme la ribonucléase

14. Kit selon l'une quelconque des revendications 11 à 13, **caractérisé en ce que** le mélange enzymatique comprend en outre une enzyme choisie dans le groupe constitué par les osidases ou carbohydrases comme la glycosidase et la galactosidase.

15. Kit selon l'une quelconque des revendications 11 à 14 **caractérisé en ce que** le mélange enzymatique est la pancréatine.

16. Kit selon l'une quelconque des revendications 11 à 15 **caractérisé en ce que** le détergent est une solution alcaline contenant des agents tensioactifs.

17. Kit selon l'une quelconque des revendications 11 à 15, **caractérisé en ce que** le détergent est une solution alcaline contenant des agents tensioactifs et un ammonium quaternaire.

18. Kit selon l'une quelconque des revendications 11 à 17 **caractérisé en ce qu'**il comporte en outre une solution d'un désinfectant comme une solution d'hypochlorite de sodium, ou d'hypochlorite de potassium.

19. Kit selon l'une quelconque des revendications 11 à 18 **caractérisé en ce qu'**il comporte en outre une solution d'un acide susceptible de dissoudre des dépôts de sels minéraux comme le carbonate de calcium.

20. Kit selon la revendication 19 **caractérisé en ce que** l'acide est choisi dans le groupe constitué par l'acide citrique, l'acide peracétique, l'acide glycolique et l'acide hydroxyacétique.

21. Composition destinée à l'élimination du biofilms **caractérisée en ce qu'**elle comprend un mélange enzymatique contenant au moins une enzyme choisie dans le groupe des protéases, au moins une enzyme choisie dans le groupe des estérases et une amylase, et un détergent dont le pH est alcalin.

22. Composition selon la revendication 21, **caractérisée en ce que** le mélange enzymatique est la pancréatine.

23. Utilisation d'un mélange enzymatique contenant au moins une enzyme choisie dans le groupe des protéases, au moins une enzyme choisie dans le groupe des estérases et une amylase, pour la préparation d'une composition destinée à l'élimination du biofilm.

24. Utilisation selon la revendication 23, **caractérisée en ce que** le mélange enzymatique est la pancréatine.

## Claims

1. Method of removing a biofilm, **characterized in that** it comprises at least the following steps, carried out simultaneously or consecutively:
a) a solution comprising an enzyme mixture containing at least one enzyme chosen from the group of proteases, at least one enzyme chosen from the group of esterases and an amylase is prepared;
b) a solution comprising a detergent with an alkaline pH is prepared; and
c) said solutions are applied, by washing or by circulation, to the surface to be treated.

2. Method according to Claim 1, **characterized in that** it furthermore comprises the following steps, carried out simultaneously or consecutively:
d) a solution comprising an acid capable of dissolving deposits of mineral salts is prepared; and
e) said solution is applied, by washing or by circulation, to the surface to be treated.

3. Method according to either of the preceding claims, **characterized in that** the enzyme chosen from the group of proteases is chosen from the group formed by exopeptidases or endopeptidases, such as trypsin.

4. Method according to any one of the preceding claims, **characterized in that** the enzyme chosen from the group of esteraes is a carboxyl ester hydrolase, such as lipase, a phospholipase and/or a phosphonodiesterase, such as ribonuclease.

5. Method according to any one of the preceding claims, **characterized in that** the enzyme mixture furthermore comprises an enzyme chosen from the group formed by osidases or carbohydrases, such as glycosidase and galactosidase.

6. Method according to any one of the preceding claims, **characterized in that** the enzyme mixture is pancreatin.

7. Method according to any one of the preceding claims, **characterized in that** the detergent is an alkaline solution containing surfactants.

8. Method according to any one of the preceding claims, **characterized in that** the detergent is an alkaline solution containing surfactants and a quaternary ammonium.

9. Method according to any one of the preceding claims, **characterized in that** the detergent solution furthermore contains a disinfectant such as a sodium hypochlorite or potassium hypochlorite solution.

10. Method according to Claim 2, **characterized in that**, in the acid for removing the deposits of mineral salts, the acid is chosen from the group formed by citric acid, peractetic acid, glycolic acid and hydroxyacetic acid.

11. Kit intended for removing a biofilm, **characterized in that** it comprises at least one solution of an enzyme mixture containing at least one enzyme chosen from the group of proteases, at least one enzyme chosen from the group of esterases and an amylase, and which comprises at least one solution of a detergent with an alkaline pH.

12. Kit according to Claim 11, **characterized in that** the enzyme chosen from the group of proteases is chosen from the group formed by exopeptidases or endopeptidases, such as trypsin.

13. Kit according to either of Claims 11 and 12, **characterized in that** the enzyme chosen from the group of esterases is a carboxyl ester hydrolase, such as lipase, a phospholipase and/or a phosphonodiesterase, such as ribonuclease.

14. Kit according to any one of Claims 11 to 13, **characterized in that** the enzyme mixture furthermore comprises an enzyme chosen from the group formed by osidases or carbohydrases, such as glycosidase and galactosidase.

15. Kit according to any one of Claims 11 to 14, **characterized in that** the enzyme mixture is pancreatin.

16. Kit according to any one of Claims 11 to 15, **characterized in that** the detergent is an alkaline solution containing surfactants.

17. Kit as claimed any one of Claims 11 to 15,
**characterized in that** the detergent is an alkaline solution containing surfactants and a quaternary ammonium.

18. Kit according to any one of Claims 11 to 17,
**characterized in that** it furthermore includes a solution of a disinfectant such as a sodium hypochlorite or potassium hypochlorite solution.

19. Kit according to any one of Claims 11 to 18,
**characterized in that** it furthermore includes a solution of an acid capable of dissolving deposits of mineral salts such as calcium carbonate.

20. Kit according to Claim 19, **characterized in that** the acid is chosen from the group formed by citric acid, peractetic acid, glycolic acid and hydroxyacetic acid.

21. Composition intended for removing a biofilm, **characterized in that** it comprises an enzyme mixture containing at least one enzyme chosen from the group of proteases, at least one enzyme chosen from the group of esterases and an amylase, and a detergent with an alkaline pH.

22. Composition according to Claim 21, **characterized in that** the enzyme mixture is pancreatin.

23. Use of an enzyme mixture containing at least one enzyme chosen from the group of proteases, at least one enzyme chosen from the group of esterases and an amylase, for the preparation of a composition intended for removing a biofilm.

24. Use according to Claim 23, **characterized in that** the enzyme mixture is pancreatin.

## Patentansprüche

1. Verfahren zur Beseitigung des Biofilms, **dadurch gekennzeichnet, dass** es mindestens die folgenden Schritte umfasst, die gleichzeitig oder nacheinander durchgeführt werden:
a) man verfügt über eine Lösung, die ein enzymatisches Gemisch umfasst, welches mindestens ein Enzym aus der Gruppe der Proteasen, mindestens ein Enzym aus der Gruppe der Esterasen und eine Amylase enthält,
b) man verfügt über eine Lösung, die ein Detergenz enthält, dessen pH alkalisch ist,
c) man bringt diese Lösungen mittels Spülung oder Umlauf auf die zu behandelnde Oberfläche auf.

2. Verfahren nach Anspruch 1, **dadurch gekennzeichnet, dass** es außerdem die folgenden Schritte umfasst, die gleichzeitig oder nacheinander durchgeführt werden:
d) man verfügt über eine Lösung, die eine Säure umfasst, welche Ablagerungen von Mineralsalzen lösen kann,
e) man bringt diese Lösung mittels Spülung oder Umlauf auf die zu behandelnde Oberfläche auf.

3. Verfahren nach einem der vorstehenden Ansprüche, **dadurch gekennzeichnet, dass** das Enzym aus der Gruppe der Proteasen aus der Gruppe ausgewählt ist, die aus Exopeptidasen oder Endopeptidasen, wie Trypsin, besteht.

4. Verfahren nach einem der vorstehenden Ansprüche, **dadurch gekennzeichnet, dass** das Enzym aus der Gruppe der Esterasen eine Carboxylester-Hydrolase, wie Lipase, eine Phospholipase und/oder eine Phosphodiesterase, wie Ribonuklease, ist.

5. Verfahren nach einem der vorstehenden Ansprüche, **dadurch gekennzeichnet, dass** das enzymatische Gemisch außerdem ein Enzym aus der Gruppe enthält, die aus Osidasen oder Carbohydrasen besteht, wie Glycosidase und Galactosidase.

6. Verfahren nach einem der vorstehenden Ansprüche, **dadurch gekennzeichnet, dass** das enzymatische Gemisch Pankreatin ist.

7. Verfahren nach einem der vorstehenden Ansprüche, **dadurch gekennzeichnet, dass** das Detergenz eine alkalische Lösung ist, die oberflächenaktive Mittel enthält.

8. Verfahren nach einem der vorstehenden Ansprüche, **dadurch gekennzeichnet, dass** das Detergenz eine alkalische Lösung ist, die oberflächenaktive Mittel und ein quaternäres Ammonium enthält.

9. Verfahren nach einem der vorstehenden Ansprüche, **dadurch gekennzeichnet, dass** die Detergenzlösung außerdem ein Desinfektionsmittel enthält, wie eine Natriumhypochlorit- oder Kaliumhypochloritlösung.

10. Verfahren nach Anspruch 2, **dadurch gekennzeichnet, dass** in der Säure zur Beseitigung der Ablagerungen von Mineralsalzen die Säure aus der Gruppe ausgewählt ist, die aus Citronensäure, Peressigsäure, Glycolsäure und Hydroxyessigsäure besteht.

11. Kit zur Beseitigung des Biofilms, **dadurch gekennzeichnet, dass** es mindestens eine Lösung aus einem enzymatischen Gemisch umfasst, welches mindestens ein Enzym aus der Gruppe der Proteasen, mindestens ein Enzym aus der Gruppe der Esterasen und eine Amylase enthält, und **dadurch**, dass es mindestens eine Lösung eines Detergenzes umfasst, dessen pH alkalisch ist.

12. Kit nach Anspruch 11, **dadurch gekennzeichnet, dass** das Enzym aus der Gruppe der Proteasen aus der Gruppe ausgewählt ist, die aus Exopeptidasen oder Endopeptidasen, wie Trypsin, besteht.

13. Kit nach einem der Ansprüche 11 oder 12, **dadurch gekennzeichnet, dass** das Enzym aus der Gruppe der Esterasen eine Carboxylester-Hydrolase, wie Lipase, eine Phospholipase und/oder eine Phosphodiesterase, wie Ribonuklease, ist.

14. Kit nach einem der Ansprüche 11 bis 13, **dadurch gekennzeichnet, dass** das enzymatische Gemisch außerdem ein Enzym aus der Gruppe enthält, die aus Osidasen oder Carbohydrasen besteht, wie Glycosidase und Galactosidase.

15. Kit nach einem der Ansprüche 11 bis 14, **dadurch gekennzeichnet, dass** das enzymatische Gemisch Pankreatin ist.

16. Kit nach einem der Ansprüche 11 bis 15, **dadurch gekennzeichnet, dass** das Detergenz eine alkalische Lösung ist, die oberflächenaktive Mittel enthält.

17. Kit nach einem der Ansprüche 11 bis 15, **dadurch gekennzeichnet, dass** das Detergenz eine alkalische Lösung ist, die oberflächenaktive Mittel und ein quaternäres Ammonium enthält.

18. Kit nach einem der Ansprüche 11 bis 17, **dadurch gekennzeichnet, dass** es außerdem eine Lösung eines Desinfektionsmittels beinhaltet, wie eine Natriumhypochlorit- oder Kaliumhypochloritlösung.

19. Kit nach einem der Ansprüche 11 bis 18, **dadurch gekennzeichnet, dass** es außerdem eine Lösung einer Säure beinhaltet, welche Ablagerungen von Mineralsalzen, wie Calciumcarbonat, lösen kann.

20. Kit nach Anspruch 19, **dadurch gekennzeichnet, dass** die Säure aus der Gruppe ausgewählt ist, die aus Citronensäure, Peressigsäure, Glycolsäure und Hydroxyessigsäure besteht.

21. Zusammensetzung zur Beseitigung des Biofilms, **dadurch gekennzeichnet, dass** sie mindestens ein enzymatisches Gemisch, welches mindestens ein Enzym aus der Gruppe der Proteasen, mindestens ein Enzym aus der Gruppe der Esterasen und eine Amylase enthält, und ein Detergenz umfasst, dessen pH alkalisch ist.

22. Zusammensetzung nach Anspruch 21, **dadurch gekennzeichnet, dass** das enzymatische Gemisch Pankreatin ist.

23. Verwendung eines enzymatischen Gemischs, das mindestens ein Enzym aus der Gruppe der Proteasen, mindestens ein Enzym aus der Gruppe der Esterasen und eine Amylase enthält, zur Herstellung einer Zusammensetzung zur Beseitigung des Biofilms.

24. Verwendung nach Anspruch 23, **dadurch gekennzeichnet, dass** das enzymatische Gemisch Pankreatin ist.
